# EUROPÄISCHE PATENTSCHRIFT

(11) **EP 2 181 120 B1**
(45) Veröffentlichungstag und Bekanntmachung des Hinweises auf die Patenterteilung: **30.03.2016**
(21) Anmeldenummer: 08774700.2
(22) Anmeldetag: 03.07.2008
(51) Int. Cl.: C07K 14/38, A61K 39/35, A61P 37/08

(54) **ALLERGENE AUS ASPERGILLUS VERSICOLOR UND VERFAHREN ZUM NACHWEIS EINER INNENRAUMBEDINGTEN SCHIMMELPILZALLERGIE**
ALLERGENS FROM ASPERGILLUS VERSICOLOR, AND METHOD OF DETECTING A MOLD ALLERGY CAUSED BY INTERIOR ROOMS
ALLERGÈNES ISSUS D'ASPERGILLUS VERSICOLOR, ET PROCÉDÉ DE DÉTERMINATION D'UNE ALLERGIE AUX MOISISSURES DES ESPACES CONFINÉS

(30) Priorität: 06.07.2007 DE 102007031947
(43) Veröffentlichungstag der Anmeldung: 05.05.2010
(73) Patentinhaber: Helmholtz-Zentrum für Umweltforschung GmbH - UFZ, 04318 Leipzig (DE)
(72) Erfinder: MÜLLER, Andrea, 04425 Taucha (DE); BENNDORF, Dirk, 04277 Leipzig (DE); BOCK, Katharina, 15236 Frankfurt (DE); VON BERGEN, Martin, 04317 Leipzig (DE); HERBARTH, Olaf, 04277 Leipzig (DE)
(74) Vertreter: Gulde & Partner
(86) Internationale Anmeldenummer: PCT/EP2008/058581
(87) Internationale Veröffentlichungsnummer: WO 2009/007298

(56) Entgegenhaltungen:
- EP-A- 1 104 768
- WO-A-2004/108765
- WO-A-2005/003310
- WO-A-2006/097539
- BENNDORF D ET AL: "Identification of spore allergens from the indoor mould Aspergillus versicolor" ALLERGY, MUNSKGAARD, COPENHAGEN, Bd. 63, Nr. 4, 1. April 2008 (2008-04-01), Seiten 454-460, XP009106522 ISSN: 0105-4538
- BOCK K ET AL: "Identification of allergens from the indoor mold Aspergillus versicolor" ALLERGY, MUNSKGAARD, COPENHAGEN, Bd. 63, Nr. suppl. 88, 11. Juni 2008 (2008-06-11), Seiten 530-531, XP009106521 ISSN: 0105-4538
- HANSEN M Y ET AL: "ALLERGENS IN APSERGILLUS FUMIGATUS: I. CHARACTERIZATION OF TWO DIFFERENT ALLERGEN EXTRACTS AND EVALUATION OF THEIR STABILITY AND THE IMPORTANCE OF CARBOHYDRATE FOR IGE BINDING" ALLERGY, MUNSKGAARD, COPENHAGEN, Bd. 49, Nr. 4, 1. Januar 1994 (1994-01-01), Seiten 235-241, XP009040600 ISSN: 0105-4538
- VERMA J ET AL: "Studies on shared antigenic/allergenic components among fungi" ALLERGY, MUNSKGAARD, COPENHAGEN, Bd. 50, Nr. 10, 1. Oktober 1995 (1995-10-01), Seiten 811-816, XP002116266 ISSN: 0105-4538
- DATABASE EMBL [Online] 30. August 2000 (2000-08-30), "Aspergillus oryzae gpdA mRNA for glyceraldehyde-3-phosphate dehydrogenase, complete cds." XP002498692 gefunden im EBI accession no. EMBL:AB032274 Database accession no. AB032274
- DATABASE UniProt [Online] 6. März 2007 (2007-03-06), "SubName: Full=Catalytic activity: R-CHOH-R' + NADP(+) <=> R-CO-R' + NADPH.; EC=<A HREF="http://srs.ebi.ac.uk/srsbin/cgi-bin/ wgetz?[enzyme-ECNumber:1.1.1.184]+-e">1.1. 1.184</A>;" XP002515546 gefunden im EBI accession no. UNIPROT:A2R5T1 Database accession no. A2R5T1
- SINGH A B ET AL: "Common environmental allergens causing respiratory allergy in India" INDIAN JOURNAL OF PEDIATRICS, ALL INDIA INSTITUTE OF MEDICAL SCIENCES, NEW DEHLI, IN, Bd. 69, Nr. 3, 1. März 2002 (2002-03-01), Seiten 245-250, XP009106528 ISSN: 0019-5456
- DATABASE UniProt [Online] 5. Juli 2005 (2005-07-05), "SubName: Full=Sorbitol/xylulose reductase Sou1-like, putative; EC=<A HREF="http://srs.ebi.ac.uk/srsbin/cgi-bin/ wgetz?[enzyme-ECNumber:1.*.*.*]+-e">1.-.-. -</A>;" XP002515547 gefunden im EBI accession no. UNIPROT:Q4WZX5 Database accession no. Q4WZX5
- RYDJORD B ET AL: "Immunoglobulin G antibodies against environmental moulds in a Norwegian healthy population shows a bimodal distribution for Aspergillus versicolor" SCANDINAVIAN JOURNAL OF IMMUNOLOGY, BLACKWELL SCIENCE PUBL., OXFORD, GB, Bd. 62, Nr. 3, 1. September 2005 (2005-09-01), Seiten 288-281, XP009106523 ISSN: 0300-9475

### SEQUENCE LISTING

<110> Helmholtz-Zentrum für Umweltforschung GmbH - UFZ
<120> Allergene aus Aspergillus versicolor und Verfahren zum Nachweis einer innenraumbedingten Schimmelpilzallergie
<130> P483907PC
<150> DE 10 2007 031 947.0
   <151> 2007-07-06
<160> 4
<170> PatentIn version 3.3
<210> 1
   <211> 336
   <212> PRT
   <213> Aspergillus versicolor
<400> 1
   Met Ala Pro Lys Val Gly Ile Asn Gly Phe Gly Arg Ile Gly Arg Ile
   1               5                   10                  15
   Val Phe Arg Asn Ala Ile Glu Ala Asp Ser Val Asp Val Ile Ala Val
               20                  25                  30
   Asn Asp Pro Phe Ile Glu Thr His Tyr Ala Ala Tyr Met Leu Lys Tyr
           35                  40                  45
   Asp Ser Gln His Gly Gln Phe Lys Gly Thr Ile Glu Thr Tyr Asp Gln
       50                  55                  60
   Gly Leu Val Val Asn Gly Lys Lys Ile Arg Phe Phe Thr Glu Arg Asp
   65                  70                  75                  80
   Pro Ala Ser Ile Pro Trp Gly Gln Ala Gly Ala Asp Tyr Ile Val Glu
                   85                  90                  95
   Ser Thr Gly Val Phe Thr Thr Gln Glu Lys Ala Gly Ala His Leu Lys
               100                 105                 110
   Gly Gly Ala Lys Lys Val Ile Ile Ser Ala Pro Ser Ala Asp Ala Pro
           115                 120                 125
   Met Phe Val Met Gly Val Asn Asn Glu Thr Tyr Lys Lys Asp Val Gln
       130                 135                 140
   Ile Leu Ser Asn Ala Ser Cys Thr Thr Asn Cys Leu Ala Pro Leu Ala
   145                 150                 155                 160
   Lys Val Ile Asn Asp Asn Phe Gly Ile Ile Glu Gly Leu Met Thr Thr
                   165                 170                 175
   Val His Ser Tyr Thr Ala Thr Gln Lys Val Val Asp Gly Pro Ser Ala
               180                 185                 190
   Lys Asp Trp Arg Gly Gly Arg Thr Ala Ala Thr Asn Ile Ile Pro Ser
           195                 200                 205
   Ser Thr Gly Ala Ala Lys Ala Val Gly Lys Val Ile Pro Ser Leu Asn
       210                 215                 220
   Gly Lys Leu Thr Gly Met Ala Met Arg Val Pro Thr Ser Asn Val Ser
   225                 230                 235                 240
   Val Val Asp Leu Thr Val Arg Thr Glu Lys Gly Val Thr Tyr Asp Gln
                   245                 250                 255
   Ile Lys Glu Ala Ile Lys Lys Ala Ser Asp Asn Glu Leu Lys Gly Ile
               260                 265                 270
   Leu Gly Tyr Thr Glu Asp Asp Ile Val Ser Thr Asp Leu Asn Gly Asp
           275                 280                 285
   Thr Arg Ser Ser Ile Phe Asp Ala Lys Ala Gly Ile Ala Leu Asn Ala
       290                 295                 300
   Asn Phe Ile Lys Leu Val Ser Trp Tyr Asp Asn Glu Trp Gly Tyr Ser
   305                 310                 315                 320
   Arg Arg Val Val Asp Leu Ile Ser Tyr Ile Ala Lys Val Asp Gly Gln
                   325                 330                 335
<210> 2
   <211> 1011
   <212> DNA
   <213> Aspergillus versicolor
<400> 2
   atggctccca aggttggaat caacggcttc ggtcgtatcg gacgtattgt cttccgtaac     60
   gccatcgagg ctgactccgt cgatgtcatc gccgtcaacg accctttcat tgagacccac    120
   tacgctgcct acatgctcaa gtatgacagt cagcacggtc agttcaaggg caccatcgag    180
   acctacgacc agggcctcgt tgtcaacggc aagaagatcc gcttcttcac cgagcgtgac    240
   cctgccagca tcccttgggg ccaggctggc gccgactaca ttgtcgagtc tactggtgtt    300
   ttcaccacgc aggagaaggc tggcgctcac ttgaagggtg gtgccaagaa ggtcatcatc     360
   tctgctcctt ccgctgatgc ccccatgttc gtcatgggcg tcaacaacga gacgtacaag     420
   aaggacgtcc aaatcctctc caacgcttct tgcaccacca actgccttgc ccctctcgcc     480
   aaggttatca acgacaactt cggtattatt gagggtctga tgacaaccgt ccactcctac     540
   actgctaccc agaaggtcgt cgatggcccc tccgccaagg actggcgtgg tggccgtacg     600
   gccgctacca acatcatccc cagctctact ggtgctgcca aggctgtcgg caaggttatt     660
   ccttcgctca acggcaagct cactggaatg gccatgcgtg ttcccacctc caacgtctct     720
   gttgttgact tgaccgtccg caccgagaag ggcgtcacct acgaccagat caaggaggcc     780
   atcaagaagg cttccgacaa cgagctcaag ggcatccttg gctacaccga ggatgacatt     840
   gtctccactg acctgaacgg tgacacccgc tcttccatct tcgatgccaa ggctggtatt     900
   gctctcaacg ccaacttcat caagctcgtt tcctggtacg acaacgagtg gggttactcc     960
   cgccgtgttg ttgacctcat ctcctacatt gccaaggtcg atggccaata g             1011
<210> 3
   <211> 266
   <212> PRT
   <213> Aspergillus versicolor
<400> 3
   Met Pro Gln Gln Ile Pro Thr Ser Ser His Leu Gln Asp Leu Phe Ser
   1               5                   10                  15
   Leu Lys Gly Lys Val Val Val Val Thr Gly Ala Ser Gly Pro Arg Gly
               20                  25                  30
   Met Gly Ile Glu Ala Ala Arg Gly Cys Ala Glu Met Gly Ala Asn Val
           35                  40                  45
   Ala Ile Thr Tyr Ser Ser Arg Pro Glu Gly Gly Glu Lys Asn Ala Glu
       50                  55                  60
   Glu Leu Ser Arg Asp Phe Gly Ile Lys Ala Lys Ala Tyr Lys Leu Asp
   65                  70                  75                  80
   Ile Gly Asn Tyr Glu Ser Val Glu Lys Leu Val Lys Asp Val Ile Ala
                   85                  90                  95
   Glu Phe Gly Gln Ile Asp Ala Phe Ile Ala Asn Ala Gly Arg Thr Ala
               100                 105                 110
   Asn Ser Gly Ile Leu Asp Gly Ser Val Lys Asp Trp Glu Glu Val Val
           115                 120                 125
   Gln Thr Asp Leu Thr Gly Thr Phe His Cys Ala Lys Ala Val Gly Pro
       130                 135                 140
   His Phe Lys Gln Arg Gly Ser Gly Ser Leu Val Ile Thr Ala Ser Met
   145                 150                 155                 160
   Ser Gly His Ile Ala Asn Phe Pro Gln Glu Gln Thr Ser Tyr Asn Val
                   165                 170                 175
   Ala Lys Ala Gly Cys Ile His Met Ala Arg Ser Leu Ala Asn Glu Trp
               180                 185                 190
   Arg Asp Phe Ala Arg Val Asn Ser Ile Ser Pro Gly Tyr Ile Asp Thr
           195                 200                 205
   Gly Leu Ser Asp Phe Val Asp Lys Lys Val Gln Asp Leu Trp Leu Ser
       210                 215                 220
   Met Ile Pro Met Gly Arg Asn Gly Asp Ala Lys Glu Leu Lys Gly Ala
   225                 230                 235                 240
   Tyr Val Tyr Leu Val Ser Asp Ala Ser Thr Tyr Thr Thr Gly Ala Asp
                   245                 250                 255
   Leu Val Ile Asp Gly Gly Tyr Thr Val Arg
               260                 265
<210> 4
   <211> 801
   <212> DNA
   <213> Aspergillus versicolor
<400> 4
   atgccgcagc aaatccccac ctcctcccac ctccaagacc tgttcagcct aaagggcaag     60
   gtcgtcgtcg tcactggtgc ctctggcccc cgtggcatgg gcattgaagc tgcccgcggc    120
   tgtgccgaaa tgggcgccaa cgttgcaatc acctactcct cccgccccga gggcggcgag    180
   aagaacgccg aagagctatc ccgcgacttc ggcatcaagg ccaaggccta caagcttgac    240
   atcggcaact acgaaagcgt agagaagcta gtgaaggatg tgatcgcaga attcggacaa    300
   attgacgctt tcatcgcgaa tgccggacgt accgcgaact cgggtatcct ggacggctca    360
   gtcaaggact gggaggaggt ggtacagacg gatttgacgg gaacgttcca ctgcgcgaag    420
   gctgtggggc cgcactttaa gcagcgcggt tcgggcagtc tcgttatcac cgccagtatg    480
   agtgggcaca ttgccaactt cccgcaagag cagacgagtt ataacgttgc gaaggctgga    540
   tgtattcaca tggcccggtc gttggcgaat gagtggaggg actttgcgcg tgtcaactcc    600
   atctcgcctg gttacattga taccgggttg agtgactttg tggataagaa ggttcaggac    660
   ctctggctta gcatgatccc tatgggccga aacggtgatg cgaaggagtt gaagggtgcc    720
   tatgtctact tggtcagtga tgccagtact tacacaactg gtgcagacct tgtcattgac    780
   ggtggataca ctgtgcggta a                                               801

## Beschreibung

Die Erfindung betrifft sieben Allergene von *Aspergillus versicolor*, die zur spezifischen Diagnostik und für die Sensibilisierung gegen diesen hauptsächlich Innenräume belastenden Schimmelpilz geeignet sind. Gegenstand der Erfindung ist auch ein immunologisches Verfahren zum Nachweis einer Sensibilisierung gegen *A. versicolor*, insbesondere einer Allergie, bei dem eines oder mehrere der genannten sieben gefundenen Allergene als diagnostische Marker eingesetzt werden. Die Erfindung betrifft weiterhin eine pharmazeutische Zusammensetzung, die eines oder mehrere dieser sieben Allergene als aktive Substanz enthält und die Verwendung eines oder mehrerer dieser sieben Allergene zur Desensibilisierung gegen *A. versicolor*.

In der heutigen Zeit wird die Gesundheit der Menschen immer mehr von Allergien beeinträchtigt. Auslöser allergischer Reaktionen können sowohl natürliche als auch künstliche Stoffe sein. Bei der Entstehung einer Allergie spielt neben der genetischen Disposition auch der sogenannte Lebensstil eine bedeutende Rolle. In den modernen Industrienationen verbringt der Mensch einen großen Teil seiner Zeit in Wohn- und Arbeitsräumen, wobei die Qualität der Innenraumluft einen wichtigen Faktor für dessen gesundheitliches Wohlbefinden darstellt. Bei fehlerhafter Bauweise und ungenügender Belüftung können sich in Innenräumen Schimmelpilze ansiedeln. Diese können die Gesundheit der Bewohner direkt beeinträchtigen, denn das Schimmelpilzwachstum führt zur Freisetzung von Schimmelpilzsporen, die in der Innenraumluft hohe Konzentration erreichen können. Neben Mykosen bei immungeschwächten Patienten oder Mykotoxikosen durch Schimmelpilztoxine werden durch die Schimmelpilze aber auch Allergien ausgelöst. Wahrscheinlich sind mehr als 5 % der deutschen Bevölkerung von einer Schimmelpilzallergie betroffen.

Als Allergene wirken dabei Proteine, die sich in den Sporen der Schimmelpilze befinden und hauptsächlich über den Atmungstrakt in den menschlichen Organismus gelangen, wo sie Atemwegsbeschwerden und Befindlichkeitsstörungen hervorrufen können. Der häufige Aufenthalt in Innenräumen und die dadurch verstärkte Exposition mit Allergenen, besonderes in der kalten Jahreszeit, begünstigt die Ausbildung einer Schimmelpilzallergie. Die Belastung durch Schimmelpilze kann einerseits durch die Untersuchung von sichtbarem Schimmel oder durch die Bestimmung der Sporenkonzentration in Luft- oder Staubproben nachgewiesen werden. Verschiedene Studien zeigten dabei, dass außer dem hochpathogenen *A. fumigatus* häufig *Aspergillus versicolor* in Innenräumen auftritt. Eine mögliche Sensibilisierung der Patienten gegen Schimmelpilze wird im Pricktest oder durch die Bestimmung spezifischer IgE bestimmt. Die bekannten diagnostischen Kits, die gegenwärtig vertrieben werden, erfassen ein relativ großes Spektrum von nahrungs- und umweltspezifischen Schimmelpilzen, nur gerade nicht die besonders innenraumrelevanten Arten.

Trotz des häufigen Auftretens von *A. versicolor* in Innenräumen ist nichts über seine Allergene bekannt und es existieren keine spezifischen Tests zum Nachweis einer Allergie gegen *A. versicolor.* In der anerkannten Allergendatenbank Allergome (www.allergome.org) sind bisher keine Allergene von *A. versicolor* hinterlegt. Es war bislang in der Fachwelt nicht bekannt, dass A. versicolor überhaupt Proteine aufweist, die sich als Allergene eignen. Im Gegenteil, Sing et al. berichtet z.B. , dass keines der Seren von 19 Personen, die in einer Bäckerei arbeiteten, keines der Seren von 53 atopischen Patienten und keines der Seren von 15 gesunden Kontrollprobanden mit einer Proteinfraktion von *A. versicolor* spezifisch reagierte, während solche Reaktionen für die anderen untersuchten Aspergillus Spezies nachweisbar waren, insbesondere für A. *fumigatus* (Singh et al., Sensitization to different species of Aspergillus in bakery workers and general atopic population, Asian Pacific Journal of Allergy and Immunology (1998) 16, 5-15). Es wurde daher bislang davon ausgegangen, dass *A. versicolor* keine geeigneten Allergene aufweist, die spezifisch sind für diese Spezies und für eine Diagnostik oder Sensibilisierung nutzbar wären. Nach allgemeiner Auffassung eignete sich *A. versicolor* nicht für die Suche nach neuen Allergenen.

Es wurden nun drei Hauptallergene und vier weitere Allergene von *A. versicolor* gefunden, die sich in den Sporen befinden und die überraschenderweise für keinen anderen Schimmelpilz als Allergene bekannt sind. Somit werden sie bisher auch nicht bei der Allergietestung auf *Aspergillus fumigatus* erfasst. Mit den sieben offenbarten Markerantigenen von *Aspergillus versicolor* ist nun eine spezifische Diagnostik von *A. versicolor* und damit eine deutliche Verbesserung der Diagnostik von Allergien gegen innenraumrelevante Schimmelpilze möglich.

Die Erfindung betrifft die in den Ansprüchen beschriebenen Gegenstände. Insbesondere betrifft die Erfindung Glyceraldehyd-3-phosphat-dehydrogenase (GAPDH) mit einem Molekulargewicht von 36.389 Dalton, einem isoelektrischen Punkt von 6,97 als Allergen von *Aspergillus versicolor.*

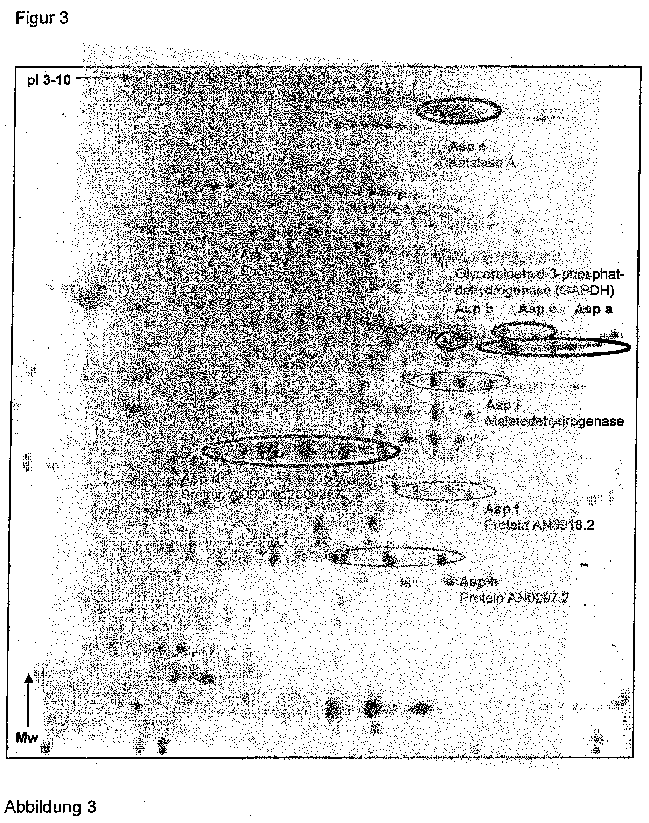

Offenbart sind die sieben gefunden Proteine gemäß Tabelle 1, und Abbildung 3. Bevorzugte Proteine sind ausgewählt aus der Gruppe umfassend eine Glyceraldehyd-3-phosphat-dehydrogenase (GAPDH; Asp a, b, c) mit der Aminosäuresequenz SEQ ID No. 1 oder Teilen davon, soweit diese Teile nicht identisch sind mit Teilen der Aminosäuresequenz der GAPDH von *Aspergillus fumigatus,* eine hypothetische Xylol/Sorbitol Reduktase mit der Aminosäuresequenz SEQ ID No. 3 oder Teilen davon, soweit diese Teile nicht identisch sind mit Teilen der Aminosäuresequenz des entsprechenden Proteins von *Aspergillus fumigatus* mit der "genebank accession number" gi/71002394/ref/XP_755878.1, eine Katalase A mit einem Molekulargewicht von 84.108 Dalton und einem isoelektrischen Punkt von 6,02 (Asp e), ein Protein mit einem Molekulargewicht von 26.713 Dalton und einem isoelektrischen Punkt von 6,53 (,Asp f), eine Enolase mit einem Molekulargewicht von 47.519 Dalton und einem isoelektrischen Punkt von 5,37 (Asp g), ein Protein mit einem Molekulargewicht von 22.007 Dalton und einem isoelektrischen Punkt von 5,85 (Asp h), eine Malatdehydrogenase mit einem Molekulargewicht von 35.764 Dalton und einem isoelektrischen Punkt von 8,82 (Asp i).

Die Proteine mit der SEQ ID No. 1 und SEQ ID No.3 weisen eine Sequenzidentität zu ihren Homologen aus *Aspergillus fumigatus* von jeweils 83% bzw. 82% auf.

Besonders bevorzugt sind Allergene von *Aspergillus versicolor* ausgewählt aus der Gruppe umfassend eine Glyceraldehyd-3-phosphat-dehydrogenase (GAPDH; Allergenbezeichnung Asp a, b, c) mit der Aminosäuresequenz SEQ ID No. 1 oder Teilen davon, soweit diese Teile nicht identisch sind mit Teilen der Aminosäuresequenz der GAPDH von *Aspergillus fumigatus,* und eine hypothetische Xylol/Sorbitol Reduktase (Protein AO090012000287; Allergenbezeichnung Asp d) mit der Aminosäuresequenz SEQ ID No. 3 oder Teilen davon, soweit diese Teile nicht identisch sind mit Teilen der Aminosäuresequenz des entsprechenden Proteins von *Aspergillus fumigatus* mit der genebank accession number gi/71002394/ref/XP_755878.1.

Der Begriff "Allergene von *Aspergillus versicolor*" umfasst im Sinne der vorliegenden Erfindung nicht nur solche Alllergene die direkt aus *A. versicolor,* dessen Sporen oder Teilen bzw. Homogenisaten davon isoliert oder gewonnen werden, sondern auch synthetisch oder gentechnisch hergestellte erfindungsgemäße Allergene.

Das erfindungsgemäße Protein und Allergen eignet sich besonders zur Verwendung als diagnostische Marker zum in-vitro-Nachweis einer Schimmelpilzallergie gegen *A. versicolor,* als Medikament und zur Desensibilisierung gegen *A. versicolor.*

Offenbart sind auch Nukleinsäuren, die mindestens eine Nukleinsäuresequenz enthalten, die für ein erfindungsgemäßes Allergen kodiert. Bevorzugte Nukleinsäuren enthalten eine Sequenz ausgewählt aus der Nukleinsäuresequenz mit der SEQ ID No. 2, die für das Allergen mit der SEQ ID No. 1 kodiert, und aus der Nukleinsäuresequenz mit der SEQ ID No. 4, die für das Allergen mit der SEQ ID No. 3 kodiert. Offenbarte Nukleinsäuren können natürliche oder künstliche Nukleinsäuren sein. Sie können aus DNA oder RNA oder Derivaten davon bestehen oder diese enthalten.

Offenbarte Nukleinsäuren umfassen auch Nukleinsäuren, die eine Nukleinsäuresequenz kodierend für eines der Allergene tragen und weitere Sequenzabschnitte beinhalten, die eine gentechnische Verbringung der Nukleinsäure in eine Zelle oder in einen Mikroorganismus erlauben, wie z.B. ein geeignetes Vektorsystem. Dem Fachmann sind geeignete Vektorsysteme sowie Techniken zur Herstellung und Klonierung offenbarter Nukleinsäuren bekannt.

Offenbart ist auch ein Verfahren zur Herstellung eines oder mehrerer der Allergene. In einem bevorzugten Verfahren kommen offenbarte Nukleinsäuren zum Einsatz. Nukleinsäuren, die für ein erfindungsgemäßes Allergen kodieren, können z.B. in ein geeignetes Vektorsystem kloniert werden, welches dann dazu verwendet wird, eine geeignete Zelle oder einen geeigneten Mikroorganismus zu transfizieren bzw. zu transformieren. Die so hergestellten genetisch veränderten Zellen oder Mikroorganismen exprimieren dann das gewünschte Allergen. Ggf. kann dieses Allergen nachfolgend isoliert und/oder gereinigt werden. Ein besonders bevorzugtes Verfahren zur Herstellung eines oder mehrerer Allergene umfasst die Schritte:
a) Einsatz einer offenbarten Nukleinsäure,
b) Transformation eines Mikroorgansimus oder Transfektion einer Zelle mit einer Nukleinsäure enthaltend eine erfindungsgemäße Nukleinsäure, und
c) ggf. Reinigung des hergestellten Allergens.

Die so hergestellten Allergene oder genetisch veränderten Zellen oder Mikroorganismen eignen sich besonders für die weiteren erfindungsgemäßen Verwendungen.

Offenbart sind genetisch veränderte Zellen oder Mikroorganismen enthaltend mindestens eine offenbarte Nukleinsäure.

Weiterer Gegenstand der Erfindung ist ein immunologisches Verfahren zum in-vitro-Nachweis einer Schimmelpilzallergie gegen *A. versicolor,* das dadurch gekennzeichnet ist, dass das gefundene Allergen eingesetzt wird, um in einer Körperflüssigkeitsprobe *A. versicolor* spezifische IgE- und/oder IgG-Antikörper durch Bindung an dieses spezifische Allergen nachzuweisen. Vorzugsweise erfolgt der erfindungsgemäße Nachweis der spezifischen IgE- und/oder IgG-Antikörper in einem Enzym- oder Radioimmunoassay, wobei in einem erfindungsgemäßen Assay bevorzugt Antikörper oder Aptamere bzw. Derivate davon zum Nachweis verwendet werden.

Besonders bevorzugt wird die Festphasentechnik des ELISA angewendet. Dazu wird das erfindungsgemäße Allergen an eine feste Phase gebunden, mit den Patientenproben, die die nachzuweisenden spezifischen IgE- und/oder IgG-Antikörper enthalten, inkubiert und die Bindung über enzym- oder fluoreszenzmarkierte Sekundärantikörper oder Sekundäraptamer gegen die Antikörper des Patienten (Anti-IgE- und/oder Anti-IgG-Antikörper) nachgewiesen. Als Detektionssystem können z. B. die Enzyme Peroxidase, alkalische Phosphatase oder Galactosidase und die entsprechenden Substrate dienen.

Selbstverständlich ist auch die Möglichkeit der Signalverstärkung über die Bindung mehrerer Sekundärantikörper oder Sekundäraptamere an die IgE/IgG-Primärantikörper gegeben. Auch ermöglicht die Verwendung von Streptavidin und biotinyliertem Marker-Enzym die Verknüpfung einer größeren Zahl Enzymmoleküle mit einem Antikörpermolekül.

Erfindungsgemäß kann es sich bei der Körperflüssigkeitsprobe um Blut, Serum, Plasma, Urin, Cerebrospinalflüssigkeit oder Saliva handeln, insbesondere werden Serumproben eingesetzt.

Die offenbarten Allergene gegen *A. versicolor* wurden bisher gewonnen und identifiziert, indem die Sporenproteine von *A. versicolor* durch ein- und zweidimensionale Elektrophorese aufgetrennt, geblottet worden und mit den im Patientenserum enthaltenen IgE-Antikörpern detektiert wurden. Die offenbarten Allergene wurden aus den 2D-Gelen ausgeschnitten und tryptisch verdaut und mittels Massenspektrometrie identifiziert. Für ihre Anwendung in Diagnostik und Therapie werden die Allergene mittels chromatographischer Methoden aus Sporenextrakten gereinigt. Die Überexpression der zu sequenzierenden Gene in *E. coli* oder *Pichia pastoris* (oder anderen Expressionsystemen) und die Kopplung an Taqs wie His-Tag oder Strep-Tag während der Expression erleichtert die Reinigung und Herstellung größerer Mengen der Allergene.

Besonders bevorzugt kommt in den erfindungsgemäßen Verfahren das Allergen gemäß Anspruch 1 zum Einsatz.

Gegenstand der vor vorliegenden Erfindung ist des Weiteren die Verwendung des erfindungsgemäßen Allergens gemäß Anspruch 1 als diagnostischer Marker zum in-vitro-Nachweis einer Schimmelpilzallergie gegen *A. versicolor.*

Die Erfindung betrifft auch eine pharmazeutische Zusammensetzung, die als aktive Komponente mindestens ein erfindungsgemäßes Allergen umfasst. Die pharmazeutische Zusammensetzung wird zur Desensibilisierung gegen *A. versicolor* angewendet. Neben der/den aktiven Komponente/n enthält die pharmazeutische Zusammensetzung konventionelle Hilfsstoffe, insbesondere Träger und/oder Adjuvantien. Die pharmazeutische Zusammensetzung der Erfindung wird vorzugsweise als Injektions- oder Infusionslösung bereitgestellt, z. B. als wässrige Lösung oder wässrige oder ölige Suspension. Die aktive(n) Komponente(n) sind in der pharmazeutischen Zusammensetzung in therapeutischer oder präventiver Menge enthalten, vorzugsweise in einer Menge von 0,05 bis 80 Gew.%, bezogen auf das Gesamtgewicht der Formulierung.

Offenbart ist auch die Verwendung des erfindungsgemäßen Allergens zur Herstellung eines Arzneimittels zur Desensibilisierung gegen *A. versicolor.* Auch die offenbarten genetisch veränderten Zellen und Mikroorganismen werden verwendet zur Herstellung eines Arzneimittels zur Desensibilisierung gegen *A. versicolor.*

Im Rahmen von Desensibilisierungsmaßnahmen kann das erfindungsgemäße Allergen verwendet werden. Bei diesem Verfahren werden Patienten, die allergisch auf Proteine aus *Aspergillus versicolor* reagieren mit niedrigen Dosen des erfindungsgemäßen Allergens in Kontakt gebracht. Bei diesem Verfahren führt die Gewöhnung an diese niedrig dosierte Exposition zu einer Abnahme der Allergiesymptome gegenüber einem erneuten Kontakt mit *A. versicolor* oder dessen Sporen.

In einem bevorzugten Verfahren zur Desensibilisierung gegen *A. versicolor,* werden Proteine eingesetzt, die ausgewählt sind aus der Gruppe umfassend eine Glyceraldehyd-3-phosphat-dehydrogenase (GAPDH; Asp a, b, c) mit der Aminosäuresequenz SEQ ID No. 1 oder Teilen davon, soweit diese Teile nicht identisch sind mit Teilen der Aminosäuresequenz der GAPDH von *Aspergillus fumigatus,* eine hypothetische Xylol/Sorbitol Reduktase mit der Aminosäuresequenz SEQ ID No. 3 oder Teilen davon, soweit diese Teile nicht identisch sind mit Teilen der Aminosäuresequenz des entsprechenden Proteins von *Aspergillus fumigatus* mit der genebank accession number gi/71002394/ref/XP_755878.1, eine Katalase A mit einem Molekulargewicht von 84.108 Dalton und einem isoelektrischen Punkt von 6,02 (Asp e), ein Protein mit einem Molekulargewicht von 26.713 Dalton und einem isoelektrischen Punkt von 6,53 (,Asp f), eine Enolase mit einem Molekulargewicht von 47.519 Dalton und einem isoelektrischen Punkt von 5,37 (Asp g), ein Protein mit einem Molekulargewicht von 22.007 Dalton und einem isoelektrischen Punkt von 5,85 (Asp h), eine Malatdehydrogenase mit einem Molekulargewicht von 35.764 Dalton und einem isoelektrischen Punkt von 8,82 (Asp i).

Nachfolgend soll die Erfindung an Ausführungsbeispielen näher erläutert werden, ohne sie darauf einzuschränken.

### Ausführungsbeispiele

### Identifizierung von Allergenen aus A. versicolor

Voraussetzung für die Identifizierung von Allergenen und den Nachweis einer Sensibilisierung der Patienten ist das Vorhandensein spezifischer IgE in Seren, die gegen Proteine von *A. versicolor* gerichtet sind. Der Nachweis der Reaktivität erfolgt auf Blotmembranen, die mit elektrophoretisch getrennten Proteinen von *A. versicolor* beladen sind. Eine Bande bzw. ein Spot wird in der nachfolgenden Immunfärbung mittels sekundärer anti-IgE-Antikörper (gekoppelt mit alkalischer Phosphatase) nachgewiesen und steht dabei jeweils für ein allergenes Protein.

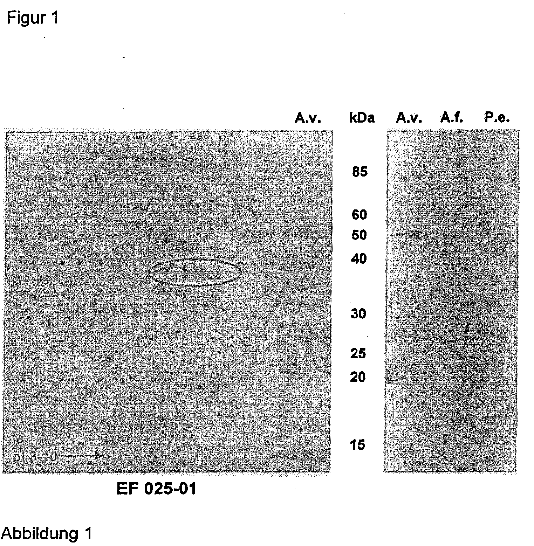

**Abbildung 1** zeigt 2D- und 1 D- Immunfärbung eines Patienten, der spezifisch auf Sporenproteine von *A. versicolor* reagiert. Die Umrandung markiert die Proteinspots, welche in der 2D- Immunfärbung am häufigsten detektiert wurden.

A.v. = Aspergillus versicolor, A.f. = Aspergillus fumigatus, P.e. = Penicilium expansum

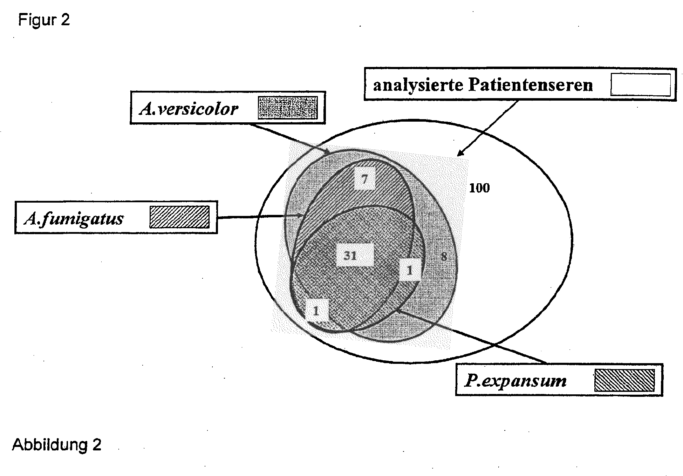

Die initiale Analyse von Patientenseren mit der 1D-Immunfärbung zeigte bei 47 Patienten von 100, die sich wegen allgemeiner allergischer Symptome in Behandlung begeben hatten, eine positive Reaktion auf die Allergenextrakte der Schimmelpilze *A. versicolor, A. fumigatus* oder *P. expansum.* Auffällig war, dass nicht ein Patientenserum exklusiv auf *A. fumigatus* oder *P. expansum* reagierte (siehe Abbildung 2). Jedoch befanden sich unter den 47 positiven Patientenseren 31 Seren, die auf alle Schimmelextrakte reagierten, während 8 Seren nur auf *A. versicolor* eine Reaktion zeigten. Diese Daten belegen, dass mit einem Test basierend auf Extrakten von *A. fumigatus* oder auf *P. expansum* nicht automatisch eine Sensibilisierung gegenüber *A. versicolor* erfasst wird.

**Abbildung 2** zeigt ein Schnittmengendiagramm zur Darstellung der Reaktivität der Patientenseren auf die Sporenproteine der Schimmelpilze *A. versicolor, A. fumigatus* und *P. expansum* in der 1D- Immunfärbung, wobei die Zahlen der Anzahl der reaktiven Seren entsprechen. Alleinige Reaktion auf *Aspergillus versicolor* (8, weiß), alleinige Reaktion auf *Aspergillus fumigatus* (0, schraffiert), alleinige Reaktion auf *Penicilium expansum* (0, Netz), Reaktion auf *Penicilium expansum* und *Aspergillus fumigatus* (1), Reaktion auf *Aspergillus versicolor* und *Penicilium expansum* (1), Reaktion auf *Aspergillus versicolor* und *Aspergillus fumigatus* (7), Reaktion auf alle drei Schimmelpilze (31), Gesamtzahl der Patienten (100, grau)

### Nachweis von Allergenen mittels Immunodetektion

Der Nachweis von Allergenen durch die Vermittlung von patienteneigenen IgE Antikörpern ist hier das Mittel zum Auffinden der Allergene und gleichzeitig der "proof of principle" für eine auf den patienteneigenen IgE Antikörpern beruhende Nachweismethode der Sensibilisierung dieser Patienten. Zunächst kann diese auch mit den hier beschriebenen Western-Blot-Verfahren nachgewiesen werden, kann aber auch auf das ELISA-Format umgestellt werden. Aber auch im ELISA-Format erfolgt die primäre Erkennung des Allergens durch die patienteneigenen IgE-Antikörper.

Diese gewonnenen Daten ermöglichten außerdem die Identifizierung von Allergenen aus Sporen von *A. versicolor.* Zur Identifizierung wurden erkannte Allergene aus 2D-Gelen ausgeschnitten, tryptisch verdaut und die Peptidmassen sowie ihre Fragmentmassen mittels Massenspektrometrie gemessen (siehe Abbildung 3, vgl. Tab. 1). Die drei erfindungsgemäßen Hauptallergene wurden bei über 70 % der analysierten Patienten gefunden und zeigten die stärkste Reaktionsintensität in der 2D-Immunfärbung. Die vier weiteren Allergene wurden ebenfalls bei mehr als 50 % der Patienten gefunden.

**Abbildung 3** zeigt die Proteine von *A. versicolor* getrennt in einem 2D- Gel nach isoelektrischem Punkt und Molekulargewicht. Tabelle 1 fasst die Charakterisierung der gefundenen Allergene zusammen.
**Tabelle 1**

**Tabelle 1**

| Allergenbezeichnung | Allergenname | Isolektrischer Punkt | Molekulargewicht (Dalton) |
|---|---|---|---|
| Asp a, b, c | Glyceraldehyd-3-phosphatdehydrogenase (GAPDH) | 6,97 | 36.389 |
| Asp d | Protein AO090012000287 | 6,11 | 28.784 |
| Asp e | Katalase A | 6,02 | 84.108 |
| Asp f | Protein AN6918.2 | 6,53 | 26.713 |
| Aspg | Enolase | 5,37 | 47.519 |
| Asp h | Protein AN0297.2 | 5,85 | 22.007 |
| Asp i | Malatdehydrogenase | 8,82 | 35.764 |

## Patentansprüche

1. Glyceraldehyd-3-phosphat-dehydrogenase (GAPDH) mit einem Molekulargewicht von 36.389 Dalton, einem isoelektrischen Punkt von 6,97 als Allergen von *Aspergillus versicolor.*

2. Verfahren zum in-vitro-Nachweis einer Schimmelpilzallergie gegen *Aspergillus versicolor,* **dadurch gekennzeichnet, dass** in einer Körperflüssigkeitsprobe *A. versicolor* spezifische IgE- und/oder IgG-Antikörper durch Bindung an das spezifische Allergen von *A. versicolor* nach Anspruch 1 nachgewiesen werden.

3. Verfahren gemäß Anspruch 2, **dadurch gekennzeichnet, dass** der Nachweis in einem Enzymimmunoassay oder Radioimmunoassay erfolgt.

4. Verfahren gemäß Anspruch 2 oder 3, **dadurch gekennzeichnet, dass** der Nachweis in einem ELISA erfolgt.

5. Verfahren gemäß einem der Ansprüche 2 bis 4, **dadurch gekennzeichnet, dass** die Körperflüssigkeit Blut, Serum, Plasma, Urin, Cerebrospinalflüssigkeit oder Saliva ist, vorzugsweise Serum.

6. Verwendung des Allergens nach Anspruch 1 als diagnostische Marker zum in-vitro-Nachweis einer Schimmelpilzallergie gegen *A. versicolor.*

7. Pharmazeutische Zusammensetzungen umfassend als aktive Komponente das Allergen nach Anspruch 1.

8. Allergen nach Anspruch 1 zur Verwendung in der Desensibilisierung gegen *A. versicolor.*

## Claims

1. Glyceraldehyde-3-phosphate dehydrogenase (GAPDH) having a molecular weight of 36,389 daltons, an isoelectric point of 6.97 as an allergen von *Aspergillus versicolor*.

2. A method for the in vitro detection of a mould allergy to *Aspergillus versicolor*, **characterized in that** *A. versicolor* specific IgE and/or IgG antibodies are detected in a sample of body fluid due to the fact that they bind to the specific allergen of *A. versicolor* according to Claim 1.

3. The method according to Claim 2, **characterized in that** detection is done in an enzyme immunoassay or a radioimmunoassay.

4. The method according to Claim 2 or 3, **characterized in that** detection is done in an ELISA.

5. The method according to any one of Claims 2 to 4, **characterized in that** the body fluid is blood, serum, plasma, urine, cerebrospinal fluid or saliva, preferably serum.

6. Use of the allergen according to Claim 1 as a diagnostic marker for the in vitro detection of a mould allergy to *A. versicolor*.

7. Pharmaceutical compositions comprising the allergen according to Claim 1 as an active component.

8. The allergen according to Claim 1 for use in the desensitization to *A. versicolor*.

## Revendications

1. Glycéraldéhyde-3-phosphate-déshydrogénase (GAPDH) avec un poids moléculaire de 36.389 daltons, un point isoélectrique de 6,97 en tant qu'allergène de *Aspergillus versicolor.*

2. Procédé de détermination in vitro d'une allergie aux moisissures en présence de *Aspergillus versicolor,* **caractérisé en ce que**, dans un échantillon de fluide corporel *A. versicolor,* des anticorps spécifiques IgE et/ou IgG sont déterminés par la liaison à l'allergène spécifique de *A. versicolor* selon la revendication 1.

3. Procédé selon la revendication 2, **caractérisé en ce que** la détermination est effectuée dans un test immuno-enzymatique ou dans un test radio-immunologique.

4. Procédé selon la revendication 2 ou 3, **caractérisé en ce que** la détermination est effectuée dans un ELISA.

5. Procédé selon l'une des revendications 2 à 4, **caractérisé en ce que** le fluide corporel est du sang, du sérum, du plasma, de l'urine, du liquide cérébrospinal ou de la salive, de préférence du sérum.

6. Utilisation de l'allergène selon la revendication 1 en tant que marqueur de diagnostic pour la détermination in vitro d'une allergie aux moisissures en présence de *A. versicolor.*

7. Compositions pharmaceutiques, comprenant en tant que composante active l'allergène selon la revendication 1.

8. Allergène selon la revendication 1, destiné à être utilisé dans la désensibilisation à A. *versicolor.*
